# EUROPEAN PATENT APPLICATION

(11) **EP 2 415 534 A1**
(43) Date of publication of application: **08.02.2012**
(21) Application number: 10008102.5
(22) Date of filing: 03.08.2010
(51) Int. Cl.: B08B 7/00, A61L 2/18, D06F 43/06

(54) **Method for cleaning and disinfecting articles**

(71) Applicant: Linde AG, 80331 München (DE)
(72) Inventor: Lindqvist, Kenneth Stig, 12838 Skarpnäck (SE); Ahlbom, Esko, 75654 Uppsala (SE)
(74) Representative: Gellner, Bernd

(57) **Abstract**

The present invention relates to a method for cleaning articles in a CO₂ cleaning machine wherein the articles are placed in a cleaning chamber of the CO₂ cleaning machine, are contacted with dense phase carbon dioxide and with an antimicrobial agent, and wherein the antimicrobial agent is neutralised inside the CO₂ cleaning machine.

## Description

The present invention relates to method for cleaning articles in a CO₂ cleaning machine comprising the following steps: placing the articles in a cleaning chamber of the CO₂ cleaning machine and contacting the articles with dense phase carbon dioxide and with an antimicrobial agent.

Dry cleaning using liquid carbon dioxide is known as an environmental friendly cleaning technique with favourable cleaning properties. Liquid carbon dioxide dry cleaning can be used to remove contaminants from garments, textiles, leather articles as well as from metal, machinery, workpieces or other parts.

A modern CO₂ cleaning machine is a quite complex machine which includes several parts and materials designed for a specific purpose and a certain task. All these parts have to withstand liquid and gaseous CO₂ as well as detergents and other chemicals which are used for cleaning. However, when strong chemicals are used it has been found difficult to design a CO₂ cleaning machine in such a way that all parts withstand these chemicals.

Dry cleaning in liquid CO₂ is an effective method for removing contaminants but it will not remove all microbial life and spores. US 6,558,622 B1 discloses a method for cleaning contaminants from articles and for microbially decontaminating the articles. This is achieved by contacting the articles with dense phase CO₂ and with an antimicrobial fluid, for example hydrogen peroxide. After the cleaning and disinfection step spent CO₂ and antimicrobial fluid are fractionated by making use of their different vapour pressures and the antimicrobial fluid is separated from the CO₂.

However, it has been found that even after the separating step antimicrobial fluid in the gaseous state is present in the gas phase of CO₂. Therefore, the antimicrobial fluid will be spread out through all CO₂ piping and CO₂ vessels of the CO₂ cleaning machine possibly causing corrosion problems.

Therefore, it is an object of the invention to provide a method for cleaning and disinfecting articles which avoids the above mentioned problems.

This object is achieved by a method for cleaning articles in a CO₂ cleaning machine comprising the following steps:
- placing the articles in a cleaning chamber of the CO₂ cleaning machine,
- contacting the articles with dense phase carbon dioxide and with an antimicrobial agent,
which is characterized in that the antimicrobial agent is neutralised inside the CO₂ cleaning machine.

The term "antimicrobial agent" shall mean any additive which acts as a sterilising or disinfecting medium. Sterilisation and disinfection shall in particular cover any process that effectively kills, eliminates or destroys all or at least a substantial portion of the microbes and microbial life, such as fungi, bacteria, viruses, and spore forms.

The term "dense phase carbon dioxide" shall mean super-critical or preferably liquid carbon dioxide. Accordingly, the present invention the process is preferably carried out using a pressure within the cleaning chamber of at least 30 bar, preferably of more than 40 bar, even more preferably of more than 50 bar. The cleaning efficiency is especially good under high pressure.

The term "CO₂ cleaning machine" shall mean a machine which is suitable for cleaning articles in dense phase carbon dioxide at enhanced pressure of at least 30 bar. The term "CO₂ cleaning machine" shall in particular comprise a cleaning machine with a cleaning chamber, a still for distilling carbon dioxide, a carbon dioxide storage tank and connecting pipes and valves. The term "CO₂ cleaning machine" shall also include a multi-machine set-up with a common storage tank and/or a common still which are connected to more than one cleaning chamber.

According to the invention the articles are cleaned in dense phase carbon dioxide and disinfected by an antimicrobial agent. By neutralizing the antimicrobial agent within the CO₂ cleaning machine no antimicrobial agent will be blown out to the atmosphere when the cleaning machine is vented. The invention provides a method for safe handling of chemicals used as antimicrobial agent. The antimicrobial agent molecules will not be vented to the atmosphere and also not be spread out in all parts of the cleaning machine and thus corrosion problems within the CO₂ cleaning machine are avoided or at least essentially reduced.

In accordance with a preferred embodiment of the invention the antimicrobial agent is neutralised within the cleaning chamber of the CO₂ cleaning machine. This will limit the exposure of the antimicrobial agent to the cleaning chamber. All other parts of the CO₂ cleaning machine will not get into contact with the antimicrobial agent. Only the cleaning chamber has to be designed to withstand the chemistry of the antimicrobial agent which is often aggressive. The risk of corrosion is considerably reduced. It is not necessary to design all parts of the CO₂ cleaning machine to withstand strong chemicals like the antimicrobial agent but only the cleaning chamber. This will reduce the costs for the cleaning machine and increase the lifetime of the machine and the so-called MTBF, Medium Time Between Failure, i.e. the downtime of the machine will be reduced.

By restricting the presence of antimicrobial agent to the cleaning chamber it is possible to only design the cleaning chamber to withstand high antimicrobial agent concentrations. All other parts of the cleaning machine do not come into contact with the antimicrobial agent and need not be re-designed or especially adapted to the impact of the antimicrobial agent. Thus, by simply improving the resistivity of the cleaning chamber against antimicrobial agent it will be possible to increase the concentration of antimicrobial agent in the cleaning chamber. For example, the solubility of H₂O₂ in liquid CO₂ is high which allows to add large amounts of H₂O₂ as antimicrobial agent into the cleaning chamber. By special design of the drum and the cleaning chamber it will also be possible to add antimicrobial agent above the solubility level in order to make sure that sufficient result is reached, i.e. the overall process could better be cost and time optimized.

Disinfection by means of the antimicrobial agent and cleaning of the articles in dense phase CO₂ can be carried out simultaneously or in sequence. In accordance with one embodiment of the invention the articles are first disinfected and then cleaned in liquid or dense phase CO₂. It is also possible to reverse this order and to first clean the articles and finally disinfect them. It is further possible to have more than one disinfection step and/or more than one cleaning step. These steps might be separate from each other or might be carried out simultaneously, at least for some period of time. For example, the articles could first be pre-cleaned in liquid CO₂, then disinfected by means of an antimicrobial agent, such as H₂O₂, which is dissolved in liquid CO₂ and finally post-cleaned in a mixture of liquid CO₂ and a detergent whereas in the beginning of the post-cleaning step still some antimicrobial agent is present which causes disinfection of the articles. Finally remaining antimicrobial agent is neutralised.

The neutralisation of the antimicrobial agent is preferably carried out by reaction with another chemical. The chemical is added to the antimicrobial agent, for example by injecting the chemical into the cleaning chamber. The chemical will react with the antimicrobial agent and neutralise it, for example by decomposing the antimicrobial agent.

In case hydrogen peroxide is used as antimicrobial agent the neutralisation can be achieved by adding chemicals which will be easily oxidised by the hydrogen peroxide. It has been found advantageous to use a neutralisation agent which consists of more than 80 % by volume, more than 90 % by volume or more than 95 % by volume of organic compounds. Organic compounds will be oxidised by the hydrogen peroxide and thereby the hydrogen peroxide will be neutralised.

The chemical which is used for neutralisation of the antimicrobial agent is preferably injected into the cleaning chamber in order to neutralise the antimicrobial agent within the cleaning chamber without prior distribution of the antimicrobial agent throughout the CO₂ cleaning machine. According to a preferred embodiment the chemical is introduced into the cleaning chamber by means of a high pressure injection pump but other methods for adding the chemical could also be used, such as by use of a compressor or by use of injection bottles.

It has been proven advantageous to use a chemical as neutralising agent which also acts as a detergent. When the disinfection has been finished the detergent is introduced into the cleaning chamber. The detergent will act in two ways: First, it will neutralise the antimicrobial agent and, second, it will act as a detergent and improve the cleaning effiency.

The detergents which are used for CO₂ cleaning application could be either a single or a blended product. The chemicals in the detergents could be different types of alcohols, esters, aldehydes etc, and in some cases it is also preferable to add water to the detergent. The total mix of detergent introduced into CO₂ cleaning machine shall be able to improve cleaning of both hydrophilic and lipophilic dirt or stains. By right choice of the detergent the neutralisation of the antimicrobial agent will be done by the detergent. For example, H₂O₂ can be neutralised by adding a detergent which comprises one or several chemicals which are easily oxidable. Several types of alcohols could for example be oxidized by H₂O₂.

According to another preferred embodiment a chemical is used as neutraliser which by reaction with the antimicrobial agent is changed to a molecule which has improved detergent performance. That means the added chemical first reacts with the antimicrobial agent and thereby neutralises the antimicrobial agent. The product of this neutralisation reaction then acts as a detergent. Example for such a chemicals are different kind of alcohols, carboxylic acids etc.

Instead of or in addition to introducing a special chemical into the CO₂ cleaning machine and to react the antimicrobial agent with that chemical it is also possible to neutralise the antimicrobial agent by means of a catalyst, for example by means of silver (Ag), manganese dioxide (MnO₂), potassium permanganate, transition metal salts, platinium etc.

It is also important that the catalyst has a large surface area. Therefore, the catalyst is preferably designed as a screen, as coated ceramic pellets or structured in the form of nano particles.

Such a catalyst can be placed in the pipeline for withdrawing gaseous CO₂ from the cleaning chamber. The stream of gaseous CO₂ leaving the cleaning chamber is passed over the catalyst and any remaining antimicrobial agent is neutralised by contact with the catalyst. It is also possible to place the catalyst in the pipeline for withdrawing gas from the still. In any case the catalytic neutralisation will remove or at least reduce antimicrobial agent in the gas phase circulating within the CO₂ cleaning machine or leaving the CO₂ cleaning machine.

Furthermore, the catalyst can be placed in a recirculation pipeline for liquid CO₂ which allows to withdraw liquid CO₂ from the cleaning chamber and then to supply the liquid CO₂ back to the cleaning chamber. Preferably, such a recirculation or round pumping system is provided with an additional filter for filtering any dirt out of the liquid CO₂. The catalyst could be integrated into the filter in order to make use of the large surface of the filter. Thereby, a large contact area for the antimicrobial agent with the catalyst is achieved and the catalytic reaction is enhanced.

In accordance with a preferred embodiment the CO₂ cleaning machine is provided with a recirculation system comprising a filter and a catalyst in series and with a bypass pipeline by-passing the catalyst. The filter can be used all the time in order to remove as much particles as possible when there is liquid CO₂ in the cleaning chamber. For simply filtering out particles from the liquid CO₂ the catalyst will be by-passed. After the disinfection procedure is finished and/or when the antimicrobial agent shall be neutralised the bypass is closed and the recirculating liquid is passed through the filter and through the catalyst. Liquid CO₂ can be withdrawn from the cleaning chamber and circulated through the filter and the catalyst and back into the cleaning chamber. Thus, the recirculating liquid is filtered and neutralised.

Preferably the antimicrobial agent is chosen from the group of
- hydrogen peroxide (H₂O₂),
- aldehydes
   - such as glutaraldehyde, chemical formula CH₂(CH₂CHO)₂ or
   - o-phthalaldehyde (ortho-phthalaldehyde), chemical formula C₆H₄(CHO)₂,
- different kinds of alcohols,
- ethylene oxide (ETO) or
- peracetic acid (CH₃CO₃H).
It is also possible to use a combination of the above mentioned antimicrobial agents, for example H₂O₂ combined with an aldehyde, an alcohol, ethylene oxide or peracetic acid.

In accordance with another preferred embodiment water is added during the disinfection phase in order to improve the disinfection. It has been shown that water will facilitate inactivation of microbes and thus assist the disinfection.

In accordance with a preferred embodiment the articles are not subjected to pure antimicrobial agent but to a mixture of antimicrobial agent and dense phase CO₂. The antimicrobial agent is either injected into, dissolved in or mixed with dense phase CO₂, preferably liquid CO₂, and then the mixture of CO₂ and antimicrobial agent is introduced into the cleaning chamber. Alternatively, the antimicrobial agent can be injected or sprayed into the cleaning chamber which is already at least partly filled with CO₂, Preferably, the liquid CO₂ in the cleaning chamber will be moved or circulated in order to further improve the mixing of CO₂ and the antimicrobial agent. In both cases it is preferred to spray or inject the antimicrobial agent in the form of small droplets in order to achieve a good mixing with or a good solution in the CO₂.

Preferably the second option is chosen: The antimicrobial agent is introduced into the dense phase CO₂ which is already within cleaning chamber. Prior to the introduction of the antimicrobial agent dense phase CO₂ is already present in the cleaning chamber. That dense phase CO₂ is preferably used to pre-clean the articles. For pre-cleaning the articles are left in dense phase CO₂, preferably in pure dense phase CO₂, for some time before the antimicrobial agent is added.

If the cleaning chamber is provided with a rotatable drum the drum is preferably rotated during the pre-cleaning phase in order to assist the pre-cleaning operation. During and after injection of the antimicrobial agent the rotation of the drum is continued to enhance the distribution of the antimicrobial agent in the cleaning chamber.

Pre-cleaning of the articles prior to disinfecting them is advantageous because any dirt which has not been removed from the articles might consume antimicrobial agent or might have another negative impact on disinfection. The pre-cleaning in liquid or dense phase CO₂ is preferably carried out by circulating the CO₂ in the cleaning chamber. This can be achieved by withdrawing CO₂ from the cleaning chamber and pumping it through a circulation pipeline back to the cleaning chamber. Preferably the circulation pipeline is provided with a filter element in order to filter out dirt.

In order to reduce the total cleaning time it is a benefit to use a detergent in the pre-cleaning step which has no impact on the subsequent disinfection step, that is the detergent shall neither reduce the disinfection strength of the antimicrobial agent nor decompose the antimicrobial agent.

It is preferred to change the pressure once or several times during the disinfection phase. By alternating the pressure in the cleaning chamber the antimicrobial agent will be pushed into and out of holes, cavities or pores of the articles to be disinfected. The pressure changes create a pressure gradient within the articles which causes a transport of antimicrobial agent into and out of the articles. Preferably the pressure is changed - increased, decreased or alternately both - at a rate of at least 1 bar per minute, more preferred at a rate of at least 5 bar per minute, even more preferred at a rate of at least 10 bar per minute. The inventive change of pressure allows to bring the antimicrobial agent into the interior of thick material or onto or into material which by its design or by its choice of material has a limited exposure to the surrounding bath of CO₂ and antimicrobial agent.

The CO₂ cleaning step and the disinfection step may be carried out in the same cleaning chamber or in different cleaning chambers. It is possible to place the articles in a first cleaning chamber and to clean the articles in a bath of dense phase CO₂ and then transfer the articles to a second cleaning chamber wherein the articles are disinfected.

As described in connection with only one cleaning chamber the order of the CO₂ cleaning step and the disinfection step may be reversed. Irrespective of the fact that there is only one cleaning chamber or more than one cleaning chamber the order of the CO₂ cleaning step or the CO₂ cleaning steps and the disinfection step or the disinfection steps can be freely chosen and combined in numerous ways and so be adapted to a broad variety of cleaning duties.

According to another preferred embodiment the cleaning chamber is evacuated after the articles have been loaded into the cleaning chamber but before the cleaning or disinfection phase has been started. Evacuation shall in particular mean to reduce the pressure in the cleaning chamber to below 10 mbar. By providing a vacuum to the cleaning chamber the articles will be dried and any water on the surface of the articles and especially water in narrow cavities of the article will evaporate. Such water could otherwise block the antimicrobial agent from penetrating into these cavities. The same applies for air which is locked in cavities or holes of the articles and which might prevent the antimicrobial agent to come into contact with the inner surface of cavities.

By vacuum pumping the cleaning chamber such air will be sucked off the cavities or holes and in a subsequent disinfection step the penetration of the antimicrobial agent into these cavities is made easier.

In general, the invention can be used to clean and disinfect different kinds of articles, such as products of metal, plastic, rubber or glass. Preferably garments and leather products are cleaned and disinfected. In particular, the present invention is suitable for cleaning and disinfecting shoes.

The following example describes a preferred embodiment of the invention step by step, namely the cleaning and disinfecting of shoes, for example of shoes made of leather. Each of the steps has certain advantages and can be used alone or in combination with the other process steps described to further improve the invention. A man skilled in the art will select those steps which are most suitable for the intended purpose and will skip the other process steps. It is definitely not necessary to make use of all of the following process steps and the process steps are preferred for, but not limited to cleaning and disinfecting of shoes.

First the shoes to be cleaned are sorted and provided with tags. The tags are chosen such that they withstand dense phase carbon dioxide as well as the antimicrobial agent which shall be used to disinfect the shoes. Further, if any detergents or other additives are used the tags should also withstand these detergents or additives.

Next the shoes are preferably attached to a movable frame. The frame is provided with special shoe holders and is especially designed for carrying the shoes.

If necessary, in particular for extremely dirty shoes, the shoes could then be pre-cleaned by a high pressure water jet and subsequently dried by means of hot air. The pre-cleaning is preferably carried out outside the CO₂ cleaning machine.

In any case, i.e. whether or not the shoes have been pre-cleaned and/or water sprayed, the frames with the attached shoes are mounted in the cleaning chamber. Preferably the cleaning chamber is provided with a rotatable drum and the frames are mounted in the rotatable drum.

Next, pure liquid carbon dioxide is introduced into the cleaning chamber and a pre-cleaning and normalisation step is carried out. Pure carbon dioxide shall mean that technically pure carbon dioxide is used and no additive has been added to the carbon dioxide. The pre-cleaning and neutralisation step aims to remove dirt from the shoes which might have a negative impact on the subsequent disinfection process. It is also possible to add a cleaning agent or a detergent in the normalisation step if the cleaning agent does not influence the disinfection process later on.

Then the liquid carbon dioxide in the cleaning chamber is preferably replaced with new pure carbon dioxide and an antimicrobial agent. Preferably hydrogen peroxide H₂O₂, is added to the carbon dioxide, preferably to achieve a concentration of H₂O₂ in the CO₂ between 1000 ppm and 5000 ppm. It is also possible to introduce the antimicrobial agent into the cleaning chamber without substituting the liquid carbon dioxide which has been used for pre-cleaning.

The liquid carbon dioxide and the antimicrobial agent are then mixed with each other by agitating the rotatable drum. The mixing can be enhanced by special baffles inside the cleaning chamber or fixed to the drum. But in general when rotating the drum the frame and the shoes cause sufficient agitation of the liquid carbon dioxide and of the antimicrobial agent so that it is not necessary to provide special baffles.

In order to achieve a deep cleaning and to improve the disinfection and sterilisation effect the pressure within the cleaning chamber is changed at a rate of at least 3 bar per minute, preferably at least 5 bar per minute. These sudden pressure changes, pressure drops or pressure rises, will cause the carbon dioxide and the antimicrobial agent to penetrate into holes and cavities of the shoes and thereby improve the cleaning and disinfection.

The disinfection or sterilisation grade is preferably determined by measurement of the concentration of the antimicrobial agent, e.g. of the H₂O₂. The original amount of antimicrobial agent introduced into the cleaning chamber and the actual concentration of the antimicrobial agent in the cleaning chamber allow to determine the level of disinfection achieved.

When a desired or pre-determined grade of disinfection or sterilisation has been achieved it is preferred to add to the carbon dioxide / antimicrobial agent mixture a neutralising agent which can also act as detergent. The addition of the neutraliser/detergent has two positive effects: First, the neutraliser/detergent improves the dissolution of dirt attached to the shoes which has not yet been removed by the pure carbon dioxide. Second, the neutraliser/detergent reacts with the antimicrobial agent and decomposes and neutralises the antimicrobial agent. For example, when H₂O₂ which has a high oxidising capacity is used as antimicrobial agent the detergent is oxidised by the H₂O₂ whereby the H₂O₂ is decomposed to water.

Next, one or more additional cleaning steps with carbon dioxide and detergent or final rinsing steps with pure liquid carbon dioxide may follow depending on the demands and on the degree of contamination of the shoes. It is possible to add some water during one or more of the cleaning steps in order to improve the cleaning performance.

In the case of shoe cleaning oil, liquid wax or a shoe polisher might be added to the carbon dioxide during the final rinse step. Such additives are preferably added by pump injection into the liquid carbon dioxide. The oil or wax penetrates into and adheres to the surface of the shoes and thereby finishes the shoes. Any surplus wax or oil could be removed from the shoes by another final rinsing in pure liquid carbon dioxide.

After having cleaned, disinfected and finished the shoes the frames are dismounted from the cleaning chamber and the shoes are removed from the frame. If necessary the shoes are dried before packing. The clean and dry shoes are preferably packed in bags or containers in a CO₂ atmosphere. That means the air within the bags or containers is replaced by gaseous CO₂ or another inert gas. This will improve the holding time of the shoes before use. Otherwise there is a certain risk that microbes or spores which have not been completely destroyed start to grow and multiply again. In order to remove or to even out any bad smell after the cleaning process it is advantageous to add an odour to the inert gas. For example, an odouring substance could be dissolved in a liquid carbon dioxide cylinder such that "odoured" gaseous carbon dioxide can be withdrawn from the cylinder and supplied to the bags or containers for storing or transporting the shoes. It is also possible to inject an odouring substance intop the cleaning chamber during the last bath of the shoes in the cleaning chamber.

## Claims

1. Method for cleaning articles in a CO₂ cleaning machine comprising the following steps:
- placing the articles in a cleaning chamber of the CO₂ cleaning machine,
- contacting the articles with dense phase carbon dioxide and with an antimicrobial agent,
**characterized in that**
the antimicrobial agent is neutralised inside the CO₂ cleaning machine.

2. Method according to claim 1 wherein said antimicrobial agent is neutralised inside said cleaning chamber.

3. Method according to claim 2 wherein said antimicrobial agent is neutralised by a chemical which is added to the antimicrobial agent.

4. Method according to claim 3 wherein said chemical is introduced into said cleaning chamber by means of an injection pump.

5. Method according to any of claims 2 to 4 wherein a detergent is introduced into said cleaning chamber and wherein said antimicrobial agent is neutralised by said detergent.

6. Method according to any of claims 1 to 5 wherein said antimicrobial agent is neutralised by means of a catalyst.

7. Method according to any of claims 1 to 6 wherein said antimicrobial agent comprises one or more of hydrogen peroxide, ozone or peracetic acid.

8. Method according to any of claims 1 to 7 wherein said articles are pre-cleaned in pure dense phase carbon dioxide prior to adding said antimicrobial agent to said articles.

9. Method according to any of claims 1 to 8 wherein the pressure in said cleaning chamber is changed at a rate of at least 1 bar per minute, preferably at a rate of 5 bar per minute, more preferably at a rate of 10 bar per minute, when the articles are in contact with said antimicrobial agent.

10. Method according to any of claims 1 to 9 for cleaning and disinfecting shoes.

11. Method according to claim 10 wherein said shoes are attached to a movable frame and wherein said frame with said shoes is mounted into the cleaning chamber.

12. Method according to any of claims 10 or 11 wherein said shoes are pre-cleaned by water prior to contacting the shoes with dense phase carbon dioxide.
